# EUROPEAN PATENT APPLICATION

(11) **EP 2 062 604 A1**
(43) Date of publication of application: **27.05.2009**
(21) Application number: 07806154.6
(22) Date of filing: 28.08.2007
(51) Int. Cl.: A61L 27/00, A61F 2/28

(54) **BONE FILLING MATERIAL AND KIT FOR THE PREPARATION OF THE SAME**

(30) Priority: 28.08.2006 JP 2006230233
(71) Applicant: National University Corporation Nagoya University, Nagoya-shi, Aichi 464-8601 (JP); Hoya Corporation, Tokyo 161-8525 (JP)
(72) Inventor: HIRATA, Hitoshi, Nagoya-shi Aichi 464-8603 (JP); HORII, Emiko, Nagoya-shi Aichi 464-8603 (JP); NAKAO, Etsuhiro, Nagoya-shi Aichi 464-8603 (JP); TAKEUCHI, Hiroyasu, Tokyo 168-0063 (JP); TAKAYAMA, Tomoji, Tokyo 173-0037 (JP); NAKASU, Masanori, Ibaraki 304-0031 (JP)
(74) Representative: Kramer, Reinhold
(86) International application number: PCT/JP2007/066676
(87) International publication number: WO 2008/026596

(57) **Abstract**

A bone filling material prepared from both a cement material for bone filling and a crimpled fibrous material, which is filled into an affected part in such a state that the crimpled fibrous material is dispersed in a cement base material consisting of the above cement material. It is preferable that the cement material contain calcium phosphate cement and the crimpled fibrous material have a mean diameter of 10 to 500 µm.

## Description

### TECHNICAL FIELD

The present invention relates to a bone filling material for medical applications which is applied to bone defects, and to a kit for preparing such a bone filling material.
The present international application claims priority from Japanese Patent Application No. 2006-230233, filed on August 28, 2006, the entire contents of which are incorporated herein by reference.

### BACKGROUND ART

In medical fields such as orthopedics, defects that have formed in the bone due to fractures, bone tumors, bone infections and the like are filled with a bone filling material, which is used to replace bone or assist in bone adhesion. Ordinary bone filling materials are composed of a cement material--generally called "bone cement"-- which is prepared primarily from poly(methyl methacrylic acid) (PMMA) and a calcium phosphate such as hydroxyapatite. For example, bone filling is typically carried out by applying a cement material prepared in the form of a paste to the affected area (bone defect) and hardening the cement material at the site of application.
A drawback of bone filling materials made of such a bone cement is that they have a relatively low mechanical strength. In a hardened state in particular, such bone filling materials have a poor elasticity and may incur failure due to stress such as bending, pulling and compression. Efforts have thus been made to enhance the mechanical strength of bone filling materials composed of such bone cements. For example, Patent Documents 1 to 4 below describe bone filling materials of increased mechanical strength which are obtained by dispersing (mixing) various fibrous substances in a cement material.

Patent Document 1: Japanese Patent Application Laid-open No. H4-314449
Patent Document 2: Japanese Patent Application Laid-open No. H6-296679
Patent Document 3: Japanese Patent Application Laid-open No. H11-267194
Patent Document 4: Japanese Patent Application Laid-open No. 2000-262608

### DISCLOSURE OF THE INVENTION

However, in conventional fiber-containing bone filling materials such as those described in the above documents, the mechanical strength is not adequately maintained over an extended period of time in circumstances where a relatively high pressure is applied to a hardened bond filling material, such as in use at the site of a fracture (spinal area) in a patient with osteoporosis who has experienced a compression fracture of the spine, thus making it difficult to stably keep the desired shape. For example, the period required for bone adhesion at the site of fracture in a patient who has suffered a compression fracture of the spine is generally from 4 to 12 weeks. Yet, in the case of the bone filling materials described in Patent Document 4 above, after these have been delivered to the affected area, the fibrous substance disappears in about 4 weeks, resulting in a loss of strength.
The present invention was conceived so as to resolve such problems with conventional bone filling materials. It is therefore an object of the invention to provide an improved bone filling material which can be properly applied (delivered) to a site subject to relatively high forces (such as the spine), and which can achieve a high strength (particularly a high failure toughness) at the site of application over an extended period of time. Another object of the invention is to provide a set of materials (a kit) for preparing such a bone filling material.

One bone filling material provided by the present invention in order to resolve the above issues is characterized by including a "bone-filling cement material" and "crimped fiber." The bone filling material of the invention is also **characterized in that,** when applied to an affected area of a patient, the crimped fiber is contained in a dispersed state within a cement matrix composed of the cement material.
It is especially preferable for at least some of the dispersed fiber to be present within the cement matrix in a mutually entangled state.
As used herein, "crimped fiber" refers to fiber which has a non-linear external shape and which, in a natural state without the application of outside forces, is randomly bent and curled in the manner of what is commonly called curly hair.
Also, "dispersed state" refers herein to the fibrous material being substantially uniformly distributed without notable unevenness within the cement matrix of the prepared bone filling material.

In the bone filling material disclosed herein, unlike in conventional bone filling materials, the crimped fiber is present in a dispersed state within the cement matrix (and may be in the form of a substance (e.g., yarn) composed of such fibers). The degree of dispersion, i.e., the crimped fiber content, is preferably such that at least some of the dispersed fiber enters into a mutually entangled state.
In a bone filling material of such a composition, the admixture of fiber that is suitably crimped (hereinafter abbreviated as crimped fiber) enables the mechanical strength (especially the failure toughness) of the cement matrix (hardened body) to be enhanced with the addition of only a very small amount of fiber; that is, without the admixture of a large amount of fiber in the cement material as in conventional continuous fiber (synthetic fiber) composed of whiskers or linear filaments. Moreover, in cases where the inventive bone filling material which uses crimped fiber has a suitably bent or curved shape, unlike in bone filling materials that contain straight fibers lacking crimps, fiber ends can be kept from protruding outward like thorns on the outside surface of the cement matrix of the bone filling material. As a result, when the bone filling material of the invention is applied to an affected area of a patient, excessive irritation of the tissue in the affected area by fiber ends protruding outward in the manner of thorns can be prevented.

In a preferred embodiment of the bone filling material, the crimped fiber has an average diameter of from 10 µm to 500 µm. Crimped fiber having such an average diameter can be easily dispersed in the cement material (i.e., in the cement matrix after hardening). A bone filling material constituted in this way is thus able to form a filling (hardened body) of high mechanical strength (especially failure toughness) in a shape that conforms to the shape of the affected area.
In another preferred embodiment of the bone filling material, the crimped fiber includes fiber having a crimped state like that of animal hair, typically wool. Because animal hair such as wool is strongly crimped, the addition of such fiber in even a small amount readily enables a complexly entangled state to form within the cement material (within the cement matrix after hardening). Therefore, by adding and dispersing a small amount of such crimped fiber in the cement material, the mechanical strength (especially the failure toughness) of the bone filling material (hardened body) can be improved.

It is also preferable for the crimped fiber to be composed of synthetic fiber that has been crimped or multicomponent synthetic fiber that develops crimps. For example, the crimped fiber may be fiber obtained by cutting synthetic fiber that has been crimped or multicomponent synthetic fiber that develops crimps to a fiber length of substantially not more than 20 mm, and preferably not more than 10 mm.
By using fiber prepared in this way, a bone filling material containing a fibrous material having an especially large degree of crimp can be provided. That is, the mechanical strength (especially the failure toughness) of the bone filling material (hardened body) may be improved with a lower amount of fiber addition. At the same time, because the content of the fibrous material can be held to a low level, the compressive strength of the bone filling material can be kept high.

Also, it is desirable for the bone filling material to contain the above-described fiber (most preferably a synthetic fiber material having a crimped state like that of wool, such as synthetic fiber which has been crimped or multicomponent synthetic fiber which develops crimps) in an amount corresponding to preferably from about 0.01 to 5 wt %, more preferably from 0.01 to 1 wt %, and most preferably from 0.01 to 0.5 wt % (e.g., from 0.05 to 0.25 wt %) of the bone-filling cement material. In the bone filling material of this embodiment, the addition of a small amount of fiber (e.g., in the case of a synthetic fibrous material having a wool-like crimped state, an amount corresponding to from 0.01 to 1 wt%, preferably from 0.01 to 0.5 wt %, and more preferably from 0.01 to 0.25 wt % (e.g., from 0.01 to 0.1 wt %), based on the bone-filling cement material, such as from about 0.05 to about 0.25 wt %, and especially from about 0.05 to about 0.1 wt %) enhances the mechanical strength (especially the failure toughness), in addition to which, owing to the low fiber content, it enables the structural density and shape stability of the cement matrix to be kept high.

It is preferable that a calcium phosphate-based cement material is contained as the above-described cement material. The invention is able in particular to enhance the mechanical strength (especially the failure toughness) of bone filling materials that contain calcium phosphate-based cement materials, thus making it possible to provide bone filling materials of such compositions.

According to another aspect, the invention also provides a combination of suitable materials (a kit) for preparing the bone filling material disclosed herein. One bone filling material preparation kit disclosed herein includes a bone-filling cement material prepared in powder form and a fibrous material used for mixture in the cement material, and composed of crimped fiber (preferably fiber crimped in the manner of wool).
With a kit constituted in this way, a bone filling material having a high mechanical strength, including a high failure toughness, can be obtained by mixing the crimped fibrous material with the powdery cement material.

According to a preferred embodiment of the kit, the crimped fiber has an average diameter in a range of from 10 µm to 50 µm. With a kit constituted in this way, a bone filling material having an improved mechanical strength (especially failure toughness) can be prepared by adding and dispersing a small amount, such as in the above-indicated weight ratio, of a crimped fibrous material to the cement material.
Moreover, as mentioned above, the fibrous material used is preferably composed of synthetic fiber that has been crimped or multicomponent synthetic fiber that develops crimp, and is more preferably composed of fiber obtained by cutting, to a fiber length of substantially not more than 20 mm, and preferably not more than 10 mm, synthetic fiber that has been crimped or multicomponent fiber that develops crimp.
According to another preferred embodiment of the kit, a calcium phosphate-based powdery cement material is contained as the cement material. This embodiment makes it possible to prepare a calcium phosphate-based bone filling material of improved mechanical strength (especially failure toughness).

According to an especially preferred embodiment, the kit further includes a liquid used for preparing a paste containing the cement material and the fibrous material. With a kit constituted in this way, a bone filling material of excellent mechanical strength can be prepared in the form of a paste.

### BRIEF DESCRIPTION OF THE DRAWINGS

FIG. 1 schematically depicts an example of procedures for preparing the bone filling material of the invention;
FIG. 2 is a photograph showing the external shapes of respective samples before a failure test;
FIG. 3 is a photograph showing the external shapes of respective samples after a failure test;
FIG. 4 is a graph showing the mechanical strength of wool fiber-containing hardened cement bodies (bone filling material) which was measured in a test example, the horizontal axis representing the crosshead stroke (mm) and the vertical axis representing the test strength (kN);
FIG. 5 is a graph showing the mechanical strength of hardened cement bodies (bone filling material) containing no wool fiber which was measured in a test example, the horizontal axis representing the crosshead stroke (mm) and the vertical axis representing the test strength (kN);
FIG. 6 is a photograph showing suitable examples of crimped fibers which may be included in the bone filling material of the invention;
FIG. 7 is a photograph showing suitable examples (in a loosely entangled state) of crimped fibers which may be included in the bone filling material of the invention;
FIG. 8 is a micrograph showing a suitable example of crimped fiber which may be included in a bone filling material of the invention;
FIG. 9 is a micrograph showing uncrimped fiber included in a bone filling material for the sake of comparison; and
FIG. 10 is a graph showing the 50% failure impact energy (J), based on a JIS standard, which was measured in a test example, the horizontal axis representing the fiber content (mixing ratio) and the vertical axis representing the calculated failure impact energy (J).

### BEST MODE FOR CARRYING OUT THE INVENTION

Preferred embodiments of the invention are described below. Matters which are not specifically mentioned in the Specification but which are necessary to working the invention (e.g., the formulation of cement materials in the bone filling material, and the means for intimately kneading together the cement material and the fibrous material) will be understood as matters of design by persons with ordinary skill in the art based on prior art in the field. The present invention can be worked based on details disclosed in the Specification and common general technical knowledge in the field.

The cement material making up the inventive bone filling material is a material which forms a cement matrix that hardens when delivered to the affected site. Use may be made of materials of various compositions which have been hitherto been employed in related applications.
For example, cement materials composed primarily of PMMA (e.g., cement materials which, in addition to PMMA, contain also barium powder, methyl methacrylate (monomer), etc.) may be used.

An example of a preferred cement material is a calcium phosphate-based cement material. Calcium phosphate is a constituent of bone, and is desirable because it also has an excellent biocompatibility. Moreover, a calcium phosphate-based cement material, when used as a component of a bone filling material-preparing kit, can be stored in solid form (typically in powder form) until hardening treatment of the sort described subsequently is carried out, and is thus advantageous also for building the inventive kit.

The calcium phosphate-based cement material may contain calcium phosphates of various chemical compositional ratios. Preferred examples include hydroxyapatite (Ca₁₀(PO₄)₆(OH)₂) and compounds capable of forming hydroxyapatite by hydrolysis. Illustrative examples include mixtures of α-tricalcium phosphate (α-Ca₃(PO)₄)₂) as the primary ingredient with another calcium phosphate-based compound as a secondary component. The latter is exemplified by α-tricalcium phosphate to which has been added hydroxyapatite, β-tricalcium phosphate (β-Ca₃(PO₄)₂), tetracalcium phosphate (Ca₄(PO₄)₂O) or calcium hydrogen phosphate (CaHPO₄·2H₂O). It is also possible to use calcium phosphate-based compounds other than those mentioned above without particular limitation, provided the combination of compounds used is one that is capable of forming hydroxyapatite or some other calcium phosphate-based cement matrix (hardened body).
Also, compounds other than the calcium phosphate-based compound serving as the primary ingredient may also be included, provided a calcium phosphate-based cement matrix (hardened body) can be obtained. For example, a compound in which some of the calcium in the calcium phosphate-based compound is substituted with another element (e.g., strontium, barium, magnesium, iron, aluminum, sodium, potassium, hydrogen) may be included. It is also possible to include a compound in which some of the PO₄ is substituted with another acid ingredient (e.g., CO₃, BO₃, SO₄, SiO₄).

The "crimped fiber" which serves as a component of the inventive bone filling material preferably has an elasticity that confers the cement matrix with enhanced mechanical strength (especially failure toughness). The degree of crimp is not subject to any particular limitation, although the crimped fiber used is preferably composed of short fibers having a fiber length of not more than 10 mm (typically about 1 to 5 mm), which fibers are non-linear and have at least one curved or bent portion formed thereon. The overall fiber may be crimped in a wavy, crescent or corkscrew shape.
A crimped state like that of animal hair (especially wool) is preferred. The use of such animal hair (wool) itself is also possible, although it is preferable to use crimped fiber that has been artificially synthesized so as to have a crimped state like that of animal hair (especially wool), such as polymer fiber which has been subjected to crimping treatment during the production process and thus imparted with the same degree of crimp as wool. Preferred examples include crimped fiber such as biodegradable or non-biodegradable polyester fiber, polylactic acid fiber or the like in which regular or random crimps have been formed by suitable crimping treatment (e.g., heat treatment using hot steam or a heater, false twisting, etc.).
Alternatively, advantageous use may be made of multicomponent fiber formed of two or more types of resin materials of differing composition. For example, bicomponent fiber (yarn) spun from two types of polymer materials having different crystalline structures is able to achieve a high degree of crimp owing to the fact that the portions formed of the respective polymer materials have mutually differing heat shrinkage ratios. To illustrate, a bicomponent fiber in which the first component is formed of a polyester resin having a given molecular structure and the second component is formed of a polyester resin having a molecular structure differing from that of the first component (e.g., in which the first component is polyethylene terephthalate and the second component is polytrimethylene terephthalate) or some other resin may be employed as the crimped fiber referred to herein. Preferred use can be made of any of the following that has been subjected to crimping treatment: polyester fibers, nylon fibers, polylactic acid fibers, polypropylene fibers, and multicomponent fibers wherein these resins (e.g., polyester, polylactic acid) serve as at least one component.
The crimped fiber, whether it is wool fiber, synthetic fiber that has been administered crimping treatment or multicomponent fiber, has excellent properties such as resilience, stretch, flexibility and durability to flexural stress. By dispersing and placing crimped fiber having such properties within the cement matrix, the mechanical strength (especially the failure toughness) of the hardened cement body can be enhanced. Moreover, by adding a suitable amount of the crimped fiber, the shape-holding ability of the hardened cement body (the ability to maintain the shape of the hardened body over an extended period of time) can be enhanced.

It is preferable for the dispersed fiber to be present in an amount such that at least some of the dispersed fiber is in a mutually entangled state. For example, it is advantageous for the crimped fiber to be present in an amount corresponding to from 0.01 to 5 wt %, preferably from 0.01 to 1 wt %, more preferably from 0.01 to 0.5 wt %, and most preferably from 0.05 to 0.25 wt %, based on the cement material (e.g., up to 1 g (such as from 0.01 g to 1 g) per 100 g of the overall amount of powdery cement material composed of several calcium phosphate-based compounds; up to 0.5 g (such as from 0.01 g to 0.5 g) per 100 g of the overall amount of powdery cement material; or up to 0.1 g (such as from 0.01 g to 0.1 g) per 100 g of the overall amount of powdery cement material). At an amount of fiber addition below this range, the desired effects may not be achieved. On the other hand, at an amount greater than this range, the density of the cement matrix may decline, which may compromise such properties as the denseness of the structure, shape stability and compressive strength.

By employing crimped fiber, even with the addition of a very small amount as indicated above, the mechanical strength (especially failure toughness) of the bone filling material (hardened body) can be enhanced. At the same time, the compressive strength of the bone filling material can be kept at a high value. Moreover, not only is the probability of fiber ends protruding from the surface of the bone filling material (hardened body) in the manner of thorns very low on account of the very small amount of fiber added, the use of crimped fiber in itself discourages the protrusion of fiber ends from the surface. It is thus possible to prevent excessive irritation by fiber ends at the affected site where the bone filling material is delivered.

The fiber shape is preferably one that facilitates dispersion within the cement matrix and enables the denseness of the matrix structure to be maintained. For example, fiber having an average diameter of from 10 µm to 500 µm, and preferably from 10 µm to 100 µm, is desirable because it readily mixes with the powdery cement material.
The average length of the fiber used is not subject to any particular limitation owing to the fact that the ease of dispersion differs with the crimped state (the degree of bending and curling) of each fiber. However, it is desirable for the average length to be generally in a range of from 0.5 mm to 30 mm, typically in a range of from 1 mm to 30 mm, preferably in a range of from 1 mm to 10 mm, and more preferably in a range of from 1 mm to 5 mm. A fibrous material in which the length of most (e.g., 80% or more) fibers falls within such a range is especially preferred. A fibrous material having too small an average length will not help to improve mechanical strength such as failure toughness, whereas a fibrous material having to large an average length will compromise the ease of handling the bone filling material and make the bone filling material difficult to deliver to the affected site.
The fibrous material admixed with the cement matrix may be crimped fiber used directly as greige yarn (i.e., in a form where the fiber alone is present). Alternatively, yarn composed of a plurality of fibers (e.g., spun yarn or filament yarn), or a loose web of fibers prior to being spun into yarn, may be used as the fibrous material.

For example, it is advantageous to use a web of short fibers (such as a mass of loosely entangled fibers) composed of a suitable amount of entangled fiber (the individual fibers being of various lengths) that has been cut at suitable intervals of from about 0.5 mm to about 10 mm while extending the individual fibers, but without disentangling and separating the fibers. Such cutting makes the crimped fibers easy to separate and also reduces the range of fluctuation in the individual lengths of crimped fibers within the web (e.g., cutting at an interval of from 1 mm to 10 mm will result in the lengths of most fibers falling within a range of about 1 mm to 30 mm), facilitating mixture with and dispersion in the cement material. It is generally desirable to cut the fibrous material so that the lengths of the individual crimped fibers within the fibrous material (e.g., a loose web of short fibers) is substantially 20 mm or less, and preferably 10 mm or less (e.g., the presence of a small amount, based on the total number of fibers, of up to 10%, and preferably up to 5%, of fibers which are more than 10 mm in length is permissible). In this way, a fibrous material having a relatively uniform fiber length can be obtained. Moreover, a fibrous material composed of crimped fiber having such a short fiber length (20 mm or less, and preferably 10 mm or less (e.g., from 1 mm to 10 mm, and preferably from 1 mm to 5 mm)) readily mixes intimately with the cement material, enabling substantially uniform dispersion within the cement matrix.
Accordingly, a kit which includes a fibrous material (a web of easy-to-separate fibers) that has been cut in this way is preferred as a kit for preparing the bone filling material of the invention.

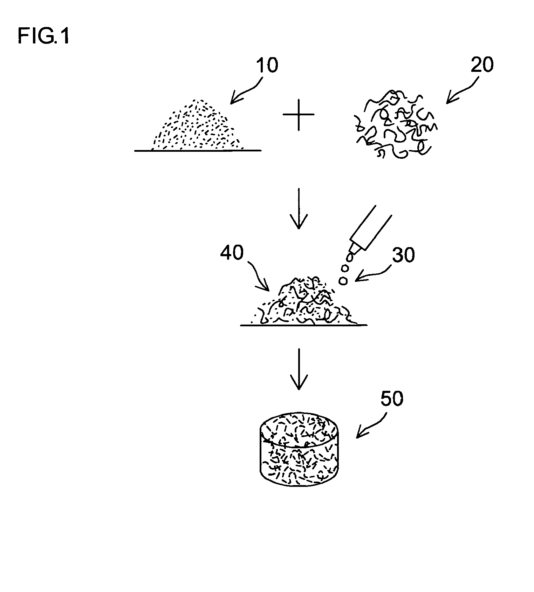

No limitation is imposed on the hardening treatment to which the bone filling material disclosed herein is subjected, so long as such treatment is carried out in accordance with the composition of the cement material. For example, as shown in FIG. 1, when a powdery calcium phosphate-based cement material 10 is used, first the powdery cement material 10 and the crimped fibrous material 20 are mixed and stirred, then a suitable amount of a liquid 30 is added to and kneaded with the stirred mixture 40. In this way, a paste-like fiber-containing cement matrix can be obtained. The liquid 30 typically includes water as the primary ingredient and may include suitable secondary ingredients. Typical secondary ingredients include hardening promoters such as disodium succinate, sodium chondroitin sulfate, sodium oxalate and sodium lactate. Antimicrobial agents, preservatives, colorants, pH regulators, salts and the like may also be included. Accordingly, a kit which includes a liquid (kneading liquid) containing water as the primary ingredient and containing also suitable amounts of several of these secondary ingredients is preferred as the kit for preparing the bone filling material of the present invention.
The amount of the liquid 30 added is not subject to any particular limitation, although the liquid is preferably added in an amount corresponding to from 10 to 100 wt % (e.g., from 10 g to 100 g per 100 g of the total amount of powdery cement material composed of a plurality of types of calcium phosphate-based compounds), based on the cement material 10.

Next, using a syringe or the like, the paste-like matrix is promptly delivered to the affected area. The paste-like matrix generally hardens in a period of from several minutes to several tens of minutes at from 30 to 37 °C, enabling a hardened cement body 50 (FIG. 1) with a shape that conforms to the affected area to be obtained.
The procedure itself for applying the bone filling material to the affected site may be the same as a conventional procedure. Because the procedure does not particularly characterize the invention, a detailed description thereof is omitted here.

The inventive bone filling material is illustrated more fully below by way of several working examples, although these examples are not intended to limit the scope of the invention.

### Preparation of Bone Filling Material (1)

A bone filling material composed of a calcium phosphate-based cement material and, as a suitable example of the crimped fiber, wool fiber was prepared in the following way.
A powdery cement material composed of 75 parts by weight of α-type tricalcium phosphate, 18 parts by weight of tetracalcium phosphate, 5 parts by weight of calcium hydrogen phosphate and 2 parts by weight of hydroxyapatite was used as the cement material. A web of merino wool fibers (average diameter, 20 µm to 40 µm) tangled together in a mass was used as the fibrous material. An aqueous solution containing 54.05 mg of sodium chondroitin sulfate and 129.72 mg of disodium succinate anhydride per mL was used as the liquid (kneading liquid).
More specifically, 60 mg of the fibrous material (merino wool fibers) was added to 12 g of the above powdery cement material. In this preparation example, the web (mass) of fibrous material prior to addition was cut at intervals of about 5 mm, thereby rendering most of the fiber into lengths falling within a given range (typically from 5 mm to 20 mm).
The above fibrous material was thoroughly mixed with the powdery cement material, following which 4 mL of the kneading liquid was added to the mixture. Thorough stirring yielded a bone cement paste in which the crimped wool fiber was uniformly dispersed. This paste was placed in a syringe and delivered into a prescribed mold.
A hardened body (Sample 1) in the shape of a disk which was slightly recessed at the center and had a diameter of 23 mm, a rim height of 5 mm and a center height of 4 mm was thus produced. Wool fibers were confirmed to be dispersed throughout the hardened body thus obtained.

### Preparation of Bone Filling Material (2)

In this preparation example, the same types of calcium phosphate-based cement material and wool fiber were used as in preparing Sample 1 above. That is, 60 mg of the above fibrous material (merino wool fibers) were added to 12 g of the above powdery cement material. However, in this production example, prior to addition, the web (mass) of fibrous material was cut at intervals of about 1 cm, thereby rendering most of the fiber into lengths falling within a given range (typically from 10 mm to 30 mm).
The above fibrous material was thoroughly mixed with the powdery cement material, following which 4 mL of the kneading liquid was added to the mixture. Thorough stirring yielded a bone cement paste in which the crimped wool fiber was uniformly dispersed. This paste was placed in a syringe and delivered into a prescribed mold.
A hardened body (Sample 2) in the shape of a disk which was slightly recessed at the center and of the same size as Sample 1 above was thus produced. Wool fibers were confirmed to be dispersed throughout the hardened body thus obtained.

### Preparation of Bone Filling Material (3)

In this preparation example, the same type of calcium phosphate-based cement material was used as in preparing Sample 1 above, but a fibrous material was not added. That is, 4 mL of the kneading liquid was added to 12 g of the powdery cement material containing no fibrous material. Thorough stirring yielded a bone cement paste. This pastes was placed in a syringe, and delivered into a prescribed mold.
A hardened body (Sample 3) in the shape of a disk which was slightly recessed at the center and of the same size as Samples 1 and 2 above was thus produced.

### Evaluation of Mechanical Strength (1)

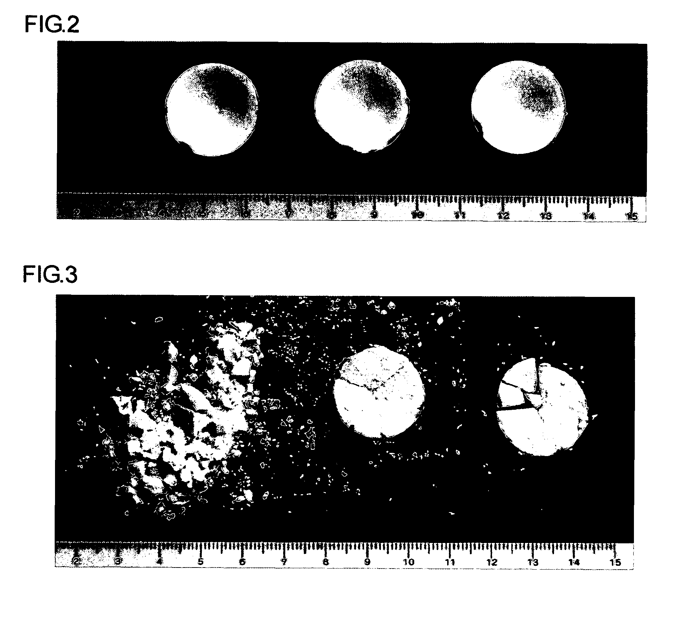

The failure tests described below were carried out on the hardened cement bodies (bone filled materials) of Samples 1 to 3 obtained as described above, and the mechanical strengths of each sample were measured. That is, a cylindrical hammer weighing 500 g and having a face with a diameter of 25 mm was dropped from a height of 10 cm onto the surface of each sample placed on a desk (see FIG. 2).

Photographs showing the condition of each sample before and after the failure test carried out as described above are presented as FIG. 2 (before test) and FIG. 3 (after test). In each photograph, Sample 3 is on the left side, Sample 1 is in the middle, and Sample 2 is on the right side. As is apparent from the photograph in FIG. 3, Sample 3 which contained no fibers was smashed to pieces in the failure test, whereas Samples 1 and 2 in which wool fibers were dispersed incurred only slight cracking (Sample 1) or partial damage (Sample 2). These test results confirmed that dispersing wool fibers in a cement matrix greatly enhances the mechanical strength (failure toughness).

### Evaluation of Mechanical Strength (2)

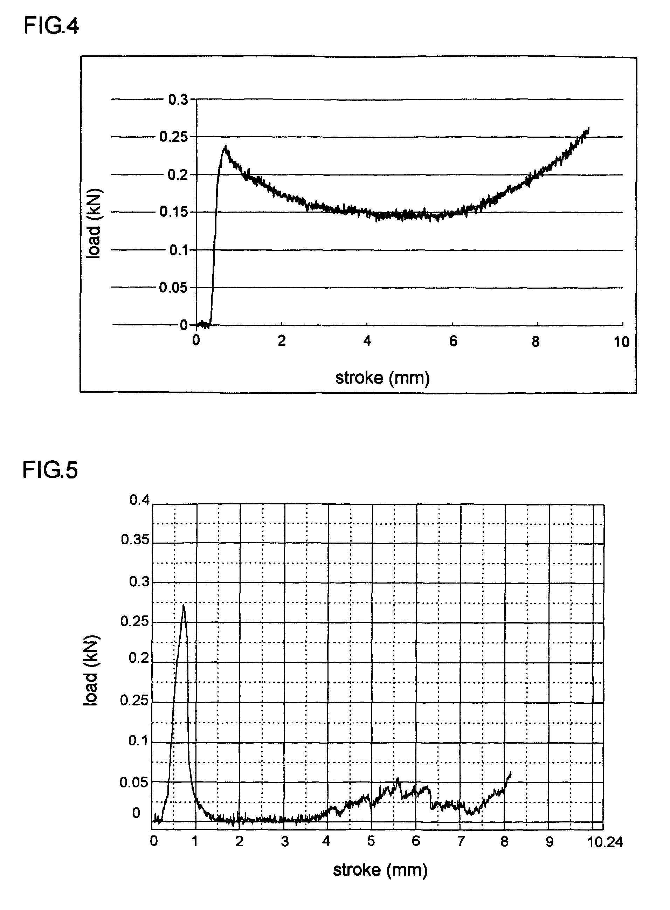

Using a wool fiber-containing bone filling material of the same composition as that used to produce above Sample 1, a cylindrical hardened body (Sample 4) having a diameter of about 10 mm and a height of about 25 mm was fabricated.
Using a fiber-free bone filling material of the same composition as that used to produce above Sample 3, a cylindrical hardened body (Sample 5) having a diameter of about 10 mm and a height of about 25 mm was fabricated.
Next, using an ordinary universal testing machine, pressure was applied to each of the above samples (cylindrical hardened bodies) by moving the crosshead downward from above at a velocity of 10 mm/min, and the change in strength at that time was examined.
Graphs of the results are shown in FIG. 4 (Sample 4) and FIG. 5 (Sample 5). In these graphs, the crosshead stroke (mm) is shown on the horizontal axis, and the load applied (kN) is shown on the vertical axis. As is apparent from these graphs, in Sample 5 containing no wool fibers, when compression was applied in excess of a limit, cracks formed and an instantaneous loss of mechanical strength occurred. On the other hand, in Sample 4 containing wool fibers, a considerable degree of mechanical strength (failure toughness) was achievable even when cracks formed under strong compression.

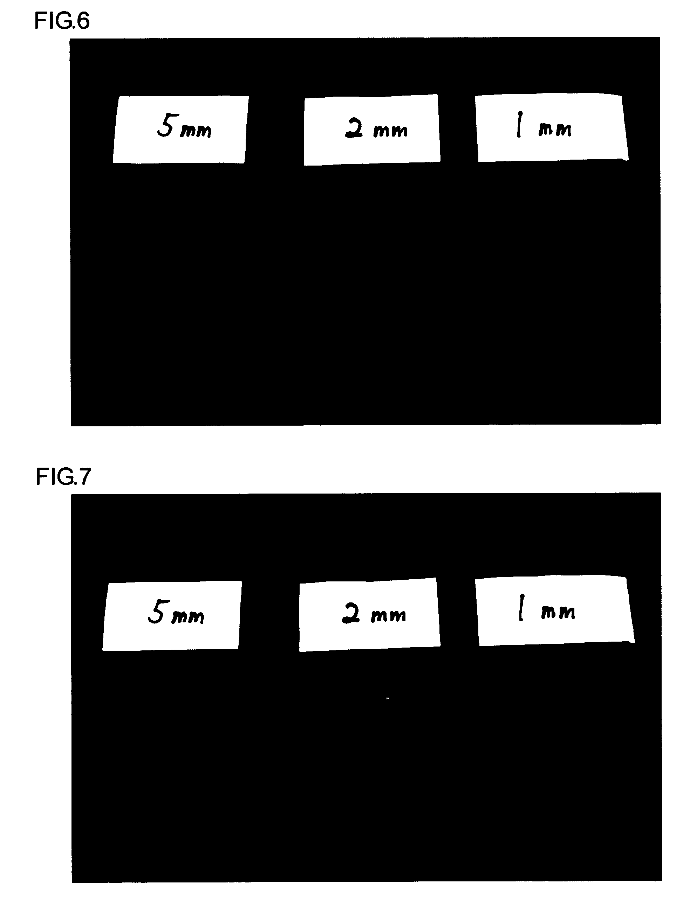

In the above-described examples, crimped fiber obtained by cutting at intervals of about 5 mm to 10 mm and having lengths of about 5 mm to 30 mm are used, although the fiber is not limited to such lengths. For example, as shown in FIG. 6, crimped fiber (the same as that used in the above examples) cut at 5 mm intervals (left side of FIG. 6), 2 mm intervals (center of FIG. 6) or 1 mm intervals (right side of FIG. 6) while being extended are also advantageous as a crimped fibrous material for carrying out the invention. These fibers cut at equal intervals are fibrous materials having a suitable degree of crimp that facilitates the formation of a loose mass of mutually entangled fibers as shown in FIG. 7 (showing loosely entangled fibers using crimped fibers cut at 5 mm intervals (left side), at 2 mm intervals (center) and at 1 mm intervals (right side)).
Although the specific data are not shown, when a sample of the same shape (i.e., a disk-shaped hardened body) as in the foregoing examples was fabricated using the above fiber cut at intervals of 2 mm (center of FIG. 6) and a failure test like that described above was carried out, the sample was found to have a high mechanical strength in the same way as Samples 1 and 2 above.

### Preparation of Bone Filling Material (4)

A bone filling material composed of a calcium phosphate-based cement material and, as a suitable example of crimped fiber, polyester fiber (crimped yarn) that had been subjected to crimping treatment was prepared as described below.
A powdery cement material obtained by mixing together 75 parts by weight of α-type tricalcium phosphate, 18 parts by weight of tetracalcium phosphate, 5 parts by weight of calcium hydrogen phosphate and 2 parts by weight of hydroxyapatite was used as the cement material. An aqueous solution containing 54.05 mg of sodium chondroitin sulfate and 129.72 mg of disodium succinate anhydrate per mL was used as the liquid (kneading liquid).

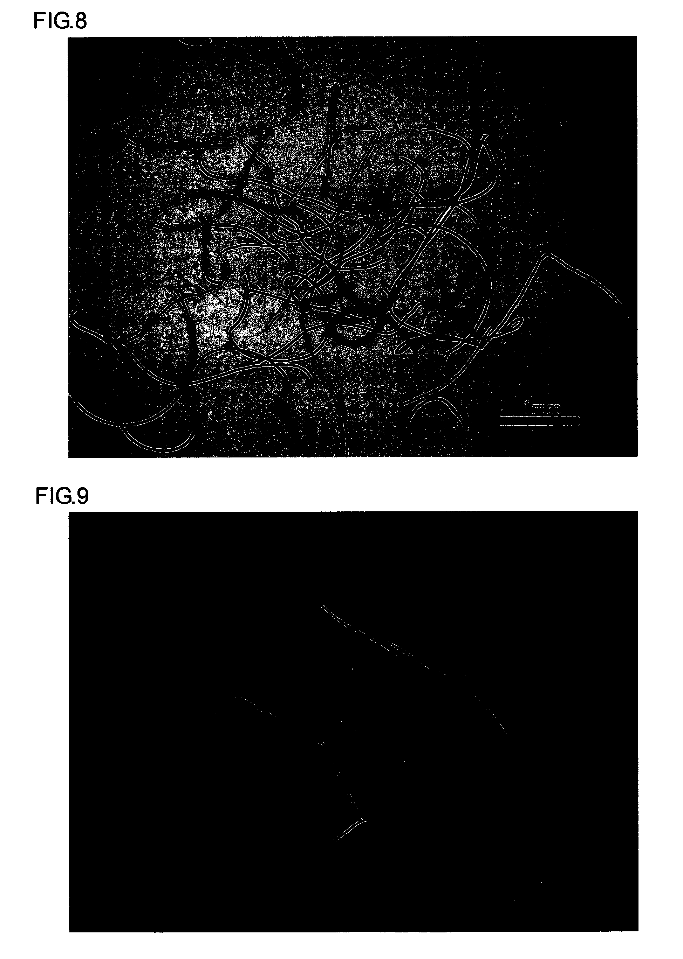

The fibrous material was a crimped polyester fibrous material having a fiber diameter of about 30 µm (examined with an optical microscope), which material is referred to below as "crimped polyester fiber." Specifically, a loose web of polyester fiber commonly sold as wadding for use in handcrafts was acquired. The product solid by Kujaku under the brand name "Shugeiwata" was purchased and used in this example.
Next, the loose web was cut in this state using scissors at intervals of about 2 mm, as measured by eye. The cut pieces were then loosened to give the fibrous material. Such cutting treatment made it possible, as can be seen in the micrograph shown in FIG. 8, for the lengths of most of the fibers to be made uniform within a given range (within a range of about 1 mm to about 5 mm).
By then adding, to 12 g of the above powdery cement material, an amount of the crimped polyester fibers corresponding to 0.0125% (about 1.5 mg), 0.05% (about 6 mg) or 0.1 % (about 12 mg) of the cement material and thoroughly mixing, a total of three types of mixed materials having the above fiber contents were prepared.
Next, 3.2 mL of the above kneading liquid was added to each of the mixtures. Three types of bone cement pastes containing uniformly dispersed crimped polyester fiber were then prepared by thorough stirring. A bone cement paste containing no polyester fiber was prepared as a control.
The resulting pastes were placed in syringes, and delivered into a prescribed mold. A plurality of disk-shaped hardened bodies having a diameter of 10 mm and a thickness (height) of 3 mm (referred to below as "crimped polyester fiber-containing test pieces") were thus produced. Substantially no crimped polyester fiber ends were found to be protruding in the manner of thorns from the surfaces of the crimped polyester fiber-containing test pieces obtained.

### Preparation of Bone Filling Material (5)

A bone filling material composed of a calcium phosphate-based cement material and uncrimped polyester fiber was prepared in the following manner as a comparative sample.
That is, a powder cement material obtained by mixing together 75 parts by weight of α-type tricalcium phosphate, 18 parts by weight of tetracalcium phosphate, 5 parts by weight of calcium hydrogen phosphate and 2 parts by weight of hydroxyapaptite was used as the cement material. In addition, an aqueous solution containing 54.05 mg of sodium chondroitin sulfate and 129.72 mg of disodium succinate anhydride per mL was used as the liquid (kneading liquid).

The fibrous material was a crimped polyester fibrous material having a fiber diameter of about 30 µm (examined with an optical microscope), which material is referred to below as "crimped polyester fiber." Specifically, a commonly sold fabric (textile) composed of polyester fiber was acquired. A plain fabric manufactured by Unitika, Ltd. was purchased and used here.
Next, the fabric was cut in this state using scissors at intervals of about 2 mm, as measured by eye. The cut pieces of fabric were then loosened to give the fibrous material. Such cutting treatment made it possible, as can be seen in the micrograph shown in FIG. 9, for the lengths of most fibers to be made uniform within a given range (within a range of about 0.5 mm to about 5 mm).
By then adding, to 12 g of the above powdery cement material, an amount of the uncrimped polyester fiber corresponding to 0.0125% (about 1.5 mg), 0.05% (about 6 mg) or 0.1 % (about 12 mg) of the cement material and thoroughly mixing, a total of three types of mixed materials having the above fiber contents were prepared.
Next, 3.2 mL of the above kneading liquid was added to each of the mixtures. Three types of bone cement pastes containing uniformly dispersed uncrimped polyester fiber were prepared by thoroughly stirring.
The resulting pastes were placed in syringes, and delivered into a prescribed mold. A plurality of disk-shaped hardened bodies having a diameter of 10 mm and a thickness (height) of 3 mm (referred to below as "uncrimped polyester fiber-containing test pieces") were thus produced. Uncrimped polyester fibers ends were found to be protruding here and there in the manner of thorns at the surface of the resulting uncrimped polyester fiber-containing test pieces.

### Evaluation of Mechanical Strength (3)

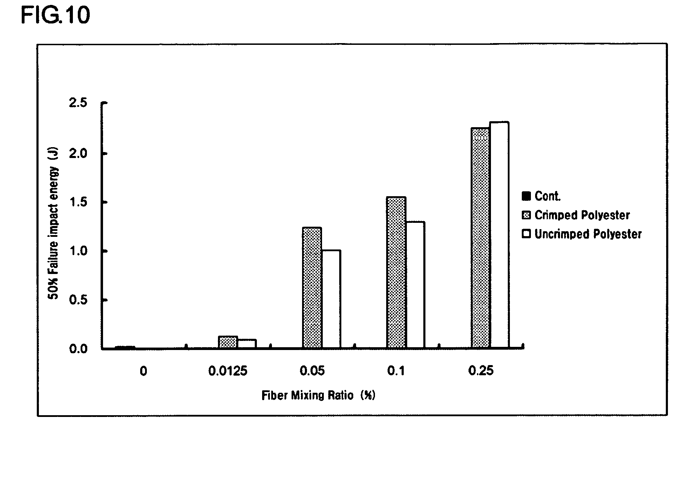

The following failure test was carried out on the hardened cement bodies (test pieces) containing crimped polyester fiber or uncrimped polyester fiber in specific contents (three types) obtained as described above, and the mechanical strength of each sample was determined.
This test was carried out in general accordance with JIS K7211: "General Rules for Testing Impact Strength of Rigid Plastics by the Falling Weight Method." Specifically, a plurality of test pieces of the shape and size indicated above were prepared for use as a total of seven types of samples (including a control) of differing fiber contents. Each of the test samples was immersed for 24 hours in an approximately 37 °C simulated body fluid (composition: Na⁺, 142.0 mM; K⁺, 5.0 mM; Mg²⁺, 1.5 mM; Ca²⁺, 2.5 mM; Cl⁻, 148.8 mM; HCO₃⁻, 4.2 mM; HPO₄²⁻, 1.0 mM; see T. Kokubo: Yogyo Kyokai Shi, 95, p. 31 (1987)), following which they were placed on a desk. Using an 8 g weight for the control test pieces, a 14 g weight for the test pieces having a fiber content of 0.0125%, and a 200 g weight for the test pieces having fiber contents of 0.05% to 0.1%, the weights were dropped from various heights onto the surface of each test piece. The 50% failure impact energy (J) at which one-half of the (20) test pieces failed was determined by the method of calculation described in JIS K7211. The results are shown in Table 1 and FIG. 10.

**Table 1. 50% Failure Impact Energy (J)**

| Fiber content | No fibers (control) | Containing crimped polyester fibers | Containing uncrimped polyester fibers | Crimp effect |
|---|---|---|---|---|
| 0% | 0.026 J | -- | -- | -- |
| 0.0125% | -- | 0.13 J | 0.09 J | 1.35 |
| 0.05% | -- | 1.24 J | 1.00 J | 1.24 |
| 0.1% | -- | 1.55 J | 1.29 J | 1.20 |

The crimp effect in Table 1 is the ratio of the 50% failure impact energy (J) for crimped polyester fiber-containing test pieces to the 50% failure impact energy (J) for uncrimped polyester fiber-containing test pieces when both types of test pieces have the same fiber content. As is apparent from Table 1 and FIG. 10, the crimped polyester fiber-containing test pieces (hardened bone filling material) also exhibited an improvement in mechanical strength (failure toughness) as the content of such fibers (from 0.0125% to 0.1%) increased. Moreover, at a fiber content (mixing ratio) within the above range (0.1% or less, such as from 0.0 1 % (0.0125%) to 0.1%, and especially from 0.05% to 0.1%), the crimped polyester fiber-containing test pieces tended to have a higher mechanical strength (failure toughness) than test pieces having the same content of uncrimped polyester fibers. Although detailed results (data) are not shown, this trend was confirmed up to a fiber content of at least 0.25% (up to 1%, depending on the type of fiber).
Hence, it was confirmed from these tests that by using crimped fiber, it is possible in particular to obtain a high mechanical strength at a low content range. Moreover, by adding a very small amount of crimped fiber as in the present test example, fiber ends can be kept from protruding in the manner of thorns from the surface of the bone filling material (hardened body), thus making it possible to effectively prevent an affected area to which the bone filling material has been delivered from being excessively irritated by the fiber ends.

Therefore, with the bone filling material of the invention, even in affected areas where more than a given amount of pressure is applied (e.g., at the site of a compression fracture of the spine in a patient with osteoporosis), for example, the mechanical strength (failure toughness) can be sustained for an extended period of time, making it possible for the shape of the bone at the affected area to be stably maintained until bone adhesion is complete.

Specific examples of the invention have been described above in detail, although these examples are provided only by way of illustration and are not intended to limit the scope of the invention. It is therefore to be understood that the invention encompasses also various modifications and changes to the above-described embodiments.

### INDUSTRIAL APPLICABILITY

The bone filling material disclosed herein is a material for application at the site of a defect that has formed in bone due to a fracture, bone tumor, bone infection or the like. Accordingly, the material of the invention is beneficial for replacing bone or assisting in bone adhesion, and for use in surgical treatment.

## Claims

1. A bone filling material comprising a bone-filling cement material and crimped fiber,
wherein when the bone filling material is applied to an affected area of a patient, the crimped fiber is contained in a dispersed state within a cement matrix composed of the cement material.

2. The bone filling material of claim 1, wherein the crimped fiber has an average diameter in a range of from 10 µm to 500 µm.

3. The bone filling material of claim 2, wherein the crimped fiber is composed of synthetic fiber that has been crimped or multicomponent fiber that develops crimp.

4. The bone filling material of claim 3, wherein the crimped fiber is composed of fiber obtained by cutting synthetic fiber that has been crimped or multicomponent synthetic fiber that develops crimp to a fiber length of substantially not more than 10 mm.

5. The bone filling material of any one of claims 1 to 4, which contains the crimped fiber in an amount corresponding to from 0.01 to I wt % of the bone-filling cement material.

6. The bone filling material of any one of claims 1 to 5, wherein a calcium phosphate-based cement material is contained as the cement material.

7. A kit for preparing a bone filling material, comprising:
a bone-filling cement material prepared in powder form, and
a fibrous material used for mixture in the cement material, and composed of crimped fiber.

8. The kit for preparing a bone filling material of claim 7, wherein the crimped fiber has an average diameter in a range of from 10 µm to 500 µm.

9. The kit for preparing a bone filling material of claim 8, wherein the fibrous material is composed of synthetic fiber that has been crimped or multicomponent synthetic fiber that develops crimp.

10. The kit for preparing a bone filling material of claim 9, wherein the fibrous material is composed of fiber obtained by cutting synthetic fiber that has been crimped or multicomponent fiber that develops crimp to a fiber length of substantially not more than 10 mm.

11. The kit for preparing a bone filling material of any one of claims 7 to 10, wherein a calcium phosphate-based powdery cement material is contained as the cement material.

12. The kit for preparing a bone filling material of claim 11, further comprising a liquid for use in preparing a paste containing the cement material and the fibrous material.
